# EUROPEAN PATENT SPECIFICATION

(11) **EP 3 967 701 B1**
(45) Date of publication and mention of the grant of the patent: **05.08.2026**
(21) Application number: 20196327.9
(22) Date of filing: 15.09.2020
(51) Int. Cl.: C07K 1/00, C07K 14/47

(54) **A METHOD OF ISOLATING MUCIN BASED ON FILTRATION STEPS**
VERFAHREN ZUR ISOLIERUNG VON MUCIN AUF DER BASIS VON FILTRATIONSSCHRITTEN
PROCÉDÉ D'ISOLATION DE MUCINE SUR LA BASE DES ÉTAPES DE FILTRATION

(43) Date of publication of application: 16.03.2022
(73) Proprietor: Technische Universität München, 80333 München (DE)
(72) Inventor: Lieleg, Oliver, 85748 Garching (DE); Marczynski, Matthias, 80331 München (DE); Winkeljann, Benjamin, 86899 Landsberg am Lech (DE)
(74) Representative: Schiweck Weinzierl Koch Patentanwälte Partnerschaft mbB

(56) References cited:
- JP-A- H05 310 799
- JP-A- H08 256 788
- US-A1- 2020 199 185
- VERONIKA J. SCHÖMIG ET AL: "An optimized purification process for porcine gastric mucin with preservation of its native functional properties", RSC ADVANCES, vol. 6, no. 50, 1 January 2016 (2016-01-01), pages 44932 - 44943, XP055655417, DOI: 10.1039/C6RA07424C

## Description

### FIELD OF THE INVENTION

The invention provides a method for isolating mucin from a mucin containing tissue whereby the mucin containing tissue is subjected to at least two subsequent filtration steps, using decreasing filter pore size diameters in each consecutive filtration step, and thereby producing a filtered mucin containing solution. Further encompassed are a bubble column and the use thereof in the method for isolating mucin.

### BACKGROUND OF THE INVENTION

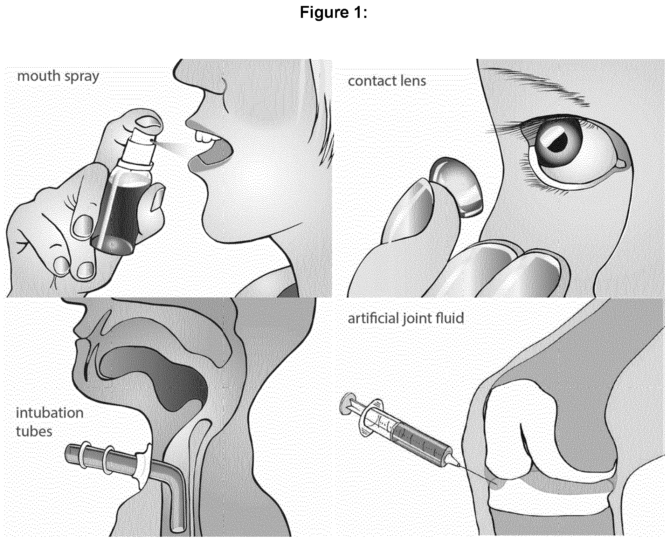

Mucins are highly glycosylated proteins, which are either membrane bound or secreted to form the structural part of mucus. For the mucus covered tissue, the mucus serves as a barrier against invading germs and mucins, which are the key component of mucus, can function as biological lubricant molecules. Thus, mucins enable for example the painless chewing and swallowing of food, or the eyelid closure. These properties make mucins invaluable for various medical applications, for example as lubricious ingredient in eye drops or mouth sprays or as germ protective lubricative for the coating of medical devices such as catheters, intubation tubes, contact lenses, or injected as artificial joint fluid in osteoarthritis therapies **(****Figure 1****).**

There are currently about 20 different types of mucins known to be found in the human body. Generally, mucins consist of a long polypeptide backbone of several thousand amino acids with various attached sugar chains, which contain negatively charged residues such as sialic acids and/or sulfates, and form the central area of the mucin molecule. Compared to the central area the outer side chains are folded and rather hydrophobic which confers the whole protein an amphiphilic character. However, mucins utilized for experimental purposes often originate from vertebrates such as pigs and cattle because the structure of porcine and bovine mucin is extremely similar to human mucin.

Commercially available mucins have been proven to have lost their lubricious properties which are, as mentioned above, crucial to any technical or experimental mucin use. This loss of properties is assumed to be due to an aggressive purification process damaging the mentioned structure of the glycoproteins. Furthermore, commercially available mucins cannot be detected by commercially available antibodies which render it in turn impossible to use commercial mucins in an experimental setup. To avoid structural damage and to preserve the mucins lubricious properties, respectively, manual purification is currently the only solution.

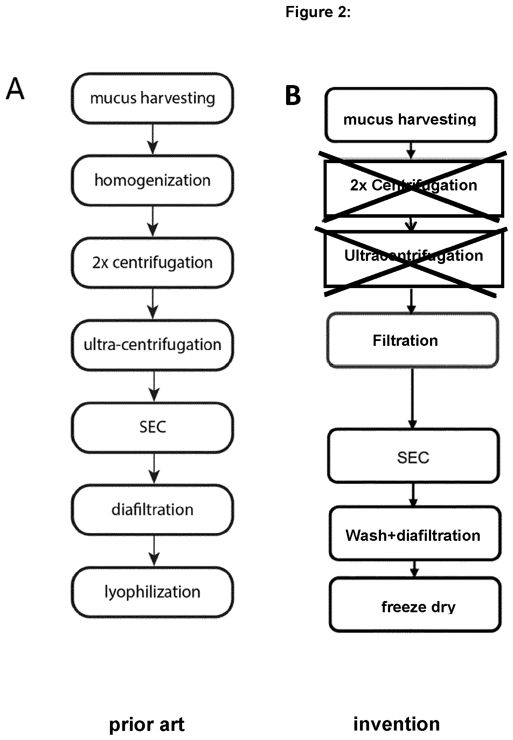

The state of the art method for manual mucin isolation is described in Schömig et al., 2016 ("An optimized purification process for porcine gastric mucin (PGM) with preservation of its native functional properties", RSC Advances 6 (50) 44932-44943). As shown in **Figure 2A****,** this method can be divided into several steps and begins with the harvesting of the mucus. For this step, a porcine stomach must be dissected and mechanically opened. To remove firmly attached food residues the tissue is rinsed under running water. While keeping the tissue on ice the mucus is collected by manually scraping the inner stomach wall with a spoon. Due to the fact that the inner stomach wall has rather strong furrows, it is relatively difficult and time-consuming to remove the mucus completely. Thus, the mucus harvest can take about a quarter of an hour of manual work per stomach. Further, the harvesting process depends on the skill of the performing person as the rinsing and mucus scraping can result in a loss of mucus and thus in a loss of mucin. After the harvesting, the collected mucus is pooled with the mucus of several other porcine stomachs. The pooled mucus is then diluted 1:5 with phosphate buffered saline (PBS), whereby the pH of the acidic stomach environment (approx. pH=2 to 4) is raised to a neutral value to reduce the activity of proteases like pepsin, which is capable of degrading the mucin termini. The mucin solution is then homogenized with a magnetic stirrer at approx. 4°C overnight. Afterwards, the mucin solution is subjected to several centrifugation steps, starting at 8300 x g for 30 min to separate and finally remove left over food residues, cell debris and other impurities. The supernatant of the centrifuged mucin solution is then again centrifuged at 15000 x g for 45 min before it is subjected to a long ultracentrifugation at 150000 x g for 60 min to further remove impurities. After a concentration step using cross-flow ultrafiltration, the mucin containing fraction is separated from smaller proteins in a size exclusion chromatography (SEC). Thereafter, the salt content of the mucin solution is reduced to a conductivity of <100µS/cm by diafiltration using distilled water. The mucin is then lyophilized for long-term storage.

As evident from the described state of the art process, the manual mucin isolation of Schömig et al., *supra,* contains many steps, whereby mucins can be lost. These steps further require a complicated logistic and time. There is thus a need for the provision of a simpler method for the isolation of mucins which also provide high yields and a good mucin quality.

The Japanese patent application JP H08 256788 A relates to the preparation of mucin during which the mucin containing solution is subjected to membrane treatment using a precision filtration membrane 0.1- 0.3um in average pore size to be concentrated. Next, the concentrated fraction is subjected to protease treatment (e.g. with trypsin) to oligomerize the mucin, and the water-soluble fraction containing the mucin thus oligomerized is concentrated through an ultrafiltration membrane. The Japanese patent application JP H05 310799 A describes purification of mucin by fine filtration and then ultrafiltration using gel membrane. A similar disclosure can be found in the US patent application US 2020/0199185 A1, which discloses methods for purifying mucin, including combining a mucin-containing substance with water and one or more purification agents.

The herein described method is thus to comply with these needs. This method is easy applicable, has a high throughput and does not require time-consuming centrifugation steps, while providing a constant mucin quality superior to commercially available mucins, as reflected by the data provided herein.

### SUMMARY OF THE INVENTION

The invention is set out in the appended set of claims.

### BRIEF DESCRIPTION OF THE DRAWINGS

The invention will be better understood with reference to the detailed description when considered in conjunction with the non-limiting examples and the drawings. It should be understood, however, that the invention, as far it is shown in the drawings, is not limited to the precise arrangements and instrumentalities of the embodiments shown in the drawings.
**Figure 1** shows examples of medical applications in which the pathogen protective and lubricious properties of mucin are useful. Mucin can, for example, be used as a lubricious ingredient in eye drops or mouth sprays. Further, mucin can be used as pathogen protective lubricative for coatings of medical advices such as contact lenses or intubation tubes. Mucin could also be injected into a joint as an artificial joint fluid and thus be used in osteoarthritis therapy.
**Figure 2** shows the comparison of the experimental steps of a manual mucin isolation according to the prior art with an illustrative embodiment of the method of mucin isolation according to the present invention; **Figure 2** **A** shows the known PGM purification of Schömig et al., *supra.* **Figure 2** **B** shows an embodiment of the present invention. In this method illustrated by **Figure 2** **B,** the mucus is separated from the surface of the stomach during incubation in an aqueous solution containing 10 mM sodium phosphate and 1 M NaCl which has an osmolarity of at least 800 mOsmol/L and thus a higher salt concentration than the salt concentration used in the prior art. The solid and liquid phases are separated, by three subsequent filtration steps to remove remaining impurities. Afterwards, a size exclusion chromatography (SEC) is performed with a buffer comprising a high salt concentration. This is followed by a subsequent washing step with a salt solution before a desalination is performed to set the conductivity to <10µS/cm. Finally, the mucin is frozen, or dried e.g. via lyophilization.

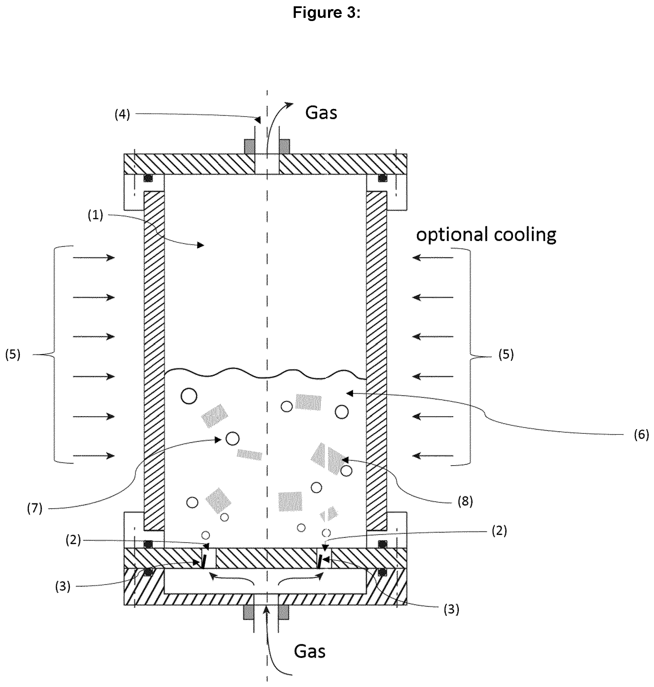

**Figure 3** shows a schematic configuration of an exemplary bubble column suitable for carrying out a method of mucin isolation of the present invention. The bubble column shown in **Figure 3** comprises a hollow chamber (1) which is sealed to the environment, an air inlet comprising of several nozzles (2) at the bottom, a non-return valve (3) ensuring that liquid cannot leak, an air outlet (4) mounted on the top and optionally a filter and a return system to prevent foam from leaking and to return the liquid to the system (not shown). The chamber (1) may optionally be equipped with an external cooling system schematically depicted by the reference arrows (5). Additionally, the chamber (1) may optionally be equipped with a control system for pH and conductivity of the liquid. Further, the chamber including all attachments may be completely demountable for easy cleaning and sterilization. The chamber (1) may also be filled with an aqueous solution (6), which is aerated by a gas whereby bubbles (7) are generated. These bubbles (7) mix the aqueous solution and the pieces of the mucin containing tissue (8) contained therein.

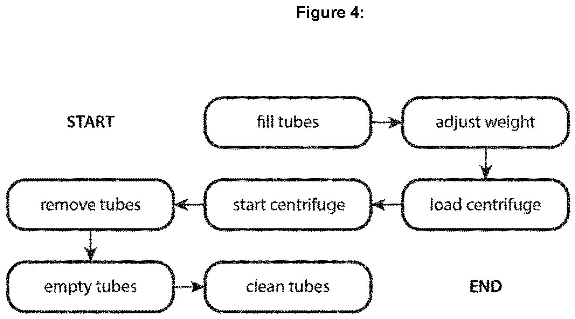

**Figure 4** shows the following manual steps during centrifugation following the protocol Schömig et al., *supra,* which are spared by the method of the present invention:
   - the filling of the tubes;
   - the taring of the filled tubes;
   - the loading of the centrifuge;
   - the starting of the centrifuge;
   - the removing of the tubes;
   - the emptying of the tubes; and
   - the cleaning of the tubes.

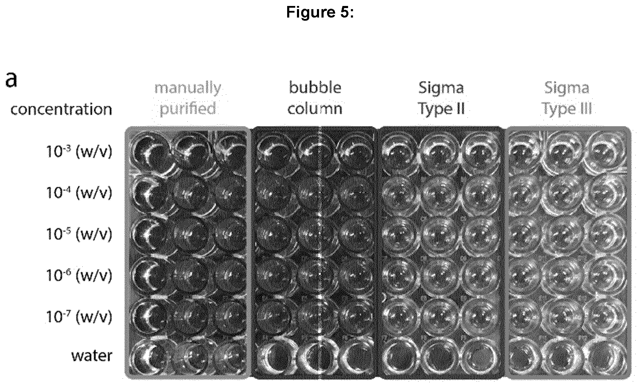

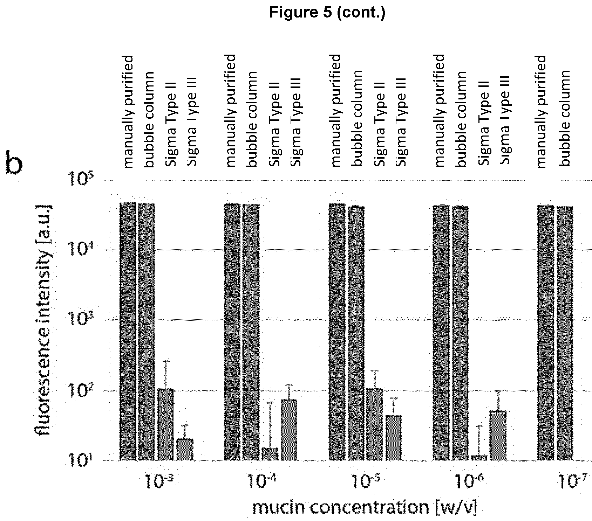

**Figure 5** shows the results of an enzyme-linked immunosorbent assay (ELISA) test for mucin detection. A mucin-specific antibody (Anti-MUC5AC) is used to detect mucin in a test solution containing mucin manually purified according to Schömig et al., a commercially available PGM (Sigma Type II and Type III) and a mucin purified according to the present invention, respectively. The anti-MUC5AC antibody exhibits a high sensitivity for manually purified mucin according to Schömig et al. as well as for mucin purified according to the present invention, whereas almost no signal for commercially available mucin can be detected.

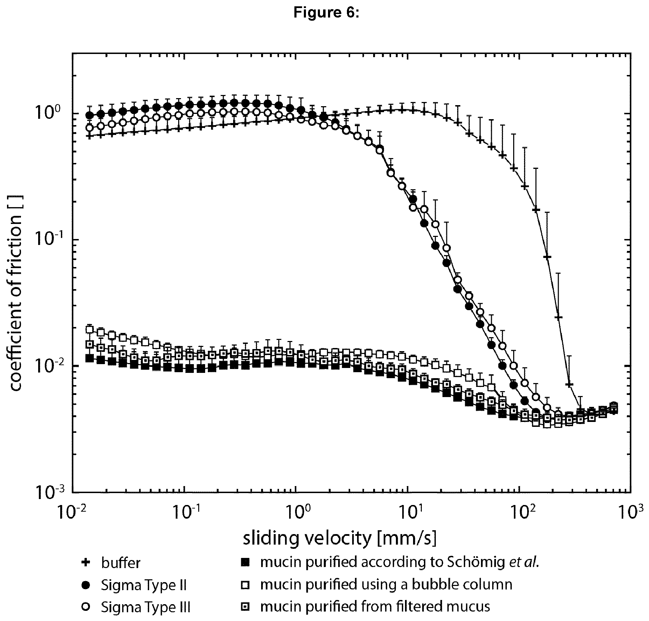

**Figure 6** shows the result of friction tests conducted with commercially available PGM (Sigma Type II and Type III), mucin purified according to Schömig et al., and two mucin variants, which have been purified by using the bubble column and the manual filtration process of shown in the present invention, respectively. The results show that the mucin variants purified according to modifications shown in the present invention yield comparable friction coefficients as mucin purified according to Schömig et al. At low sliding velocities, the coefficient of friction is about 100 times lower than that obtained for commercially available mucins.

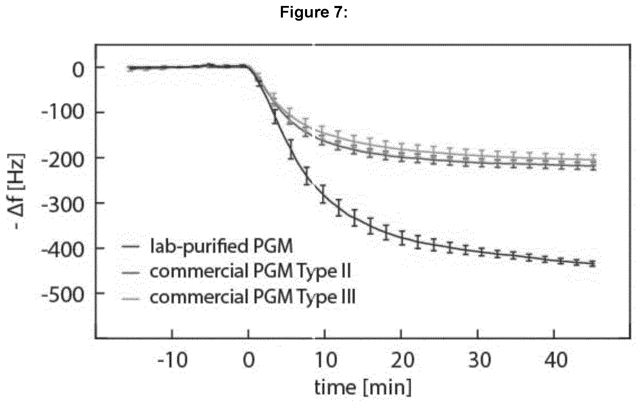

**Figure 7** shows the adsorption of the various mucins onto hydrophobic silicone. With the help of QCM measurements, the adsorption efficiency of mucin molecules on hydrophobic silicone can be measured. Mucin purified in the laboratory adsorbs significantly better on these surfaces (Δf ~ 400 Hz) than the two commercially available mucin variants (each Δf ~ 200 Hz).

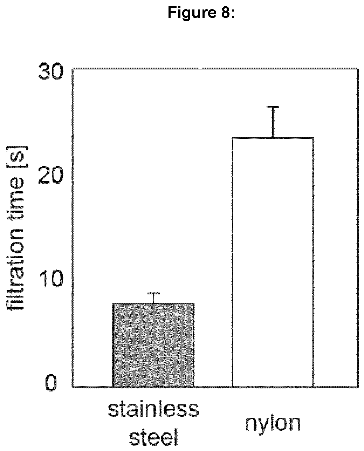

**Figure 8** shows the time required to obtain a filtrate volume of 20 mL when membranes made from stainless steel (grey bar) or nylon (white bar) are used.

### DETAILED DESCRIPTION OF THE INVENTION

As explained above, the invention is directed to method of isolating mucin from a mucin containing tissue, wherein the method comprises subjecting a mucin containing solution to at least two subsequent filtration steps, wherein the mucin containing solution has been produced by contacting a mucin containing tissue with an aqueous solution over a suitable period of time, which is at least about 10 min, at a suitable temperature, which is in the range of about 0°C to about 42°C, for transferring the mucin into the aqueous solution, wherein the aqueous solution has an osmolarity of at least about 800 mOsm/L, prior to the step of contacting the mucin containing tissue with the aqueous solution, a pore size diameter of a first filter used in a first step and a pore size diameter of a second filter used in a second step are different, and wherein the second filter used in the second step has a smaller pore size diameter than the first filter used in the first step, wherein the mucin containing solution is filtered in the first filtration step by using the first filter having the pore size diameter in the range of about 3 mm to about 0.5 mm, wherein the mucin containing solution is filtered in the second filtration step by using the pore size diameter in the range of about 600 µm to about 10 µm, thereby producing a filtered mucin containing solution. The method also comprises performing a size exclusion chromatography with the filtered mucin containing solution, thereby producing a mucin enriched solution, diafiltrating the mucin enriched solution with demineralized, distilled or ultrapure water and optionally freeze-drying the mucin enriched solution, thereby isolating the mucin.

The inventors were able to simplify and further develop a previously described method for isolating mucin from a mucin containing tissue as described by Schömig et al., 2016. For example, the overnight homogenization as described for the method of Schömig et al.,2016 are also no longer necessary. Further, the method of the present invention reduces complicated logistics and renders an in-between storage of intermediate products unnecessary. Another main advantage of the herein presented method is the use of an aqueous solution comprising a high salt concentration which allows the filtration of the mucin containing solution and eliminates all the time-consuming centrifugation steps. Further comprised is an optimized process (not part of the invention) of contacting the mucin containing tissue with the aqueous solution by using an aerated bubble column as described herein which leads to a simple and more effective isolation of mucin from a mucin containing tissue. The herein presented method is thus more time efficient and less susceptible to errors in comparison to the method of Schömig et al., *supra* and provides reproducible results which lead to a constant mucin quality and high mucin yields of about 200-300 mg per stomach per run.

In addition, the method of the invention is easy scalable thus allows the production on an industrial scale. Further, the method of the present invention is applicable to a huge variety of mucin and thus provides for a time-efficient and effective isolation of mucins. The method is thereby especially suitable for the isolation and purification of secreted mucins such as MUC2, MUC5AC, MUC5B, MUC6 und MUC7.

### Mucin containing tissues

In the present invention, any mucin containing tissue can be used. When used in the present invention the term "mucin containing tissue" refers to tissue with membrane bound or secreted mucins which have not been removed or extracted. Included are thus, for example, the tissue and mucus of a mucous membrane, if not indicated otherwise.

The mucin containing tissue may, for example, be of plant origin. For example, the mucin containing tissue may be a whole plant or parts thereof. An example for such a plant is a carnivorous plant such as a flypaper trap like *Pinguicula conzatti* or a great sundrew like *Drosera anglica,* which use mucilage secreted on the leaves to catch prey. In another embodiment the plant may be selected from the group consisting of a carnivorous plant, a marshmallow root or leaf, a forest mallow flower, a black mallow flower, a flax seeds, a fenugreek seed, a mullein flower, a coltsfoot leaf, an Icelandic lichen, a psyllium seed, a quince seed, a tuber of an orchid, a red seaweed, and red algae.

Alternatively, the mucin containing tissue may be of animal origin. The tissue may, for example, be obtained from a mammal such as a farm animal including, but not limited to, a pig, a cow, a sheep, a horse, a goat, a donkey, a dog, a cat, a rabbit or poultry such as a bird, a chicken, a turkey, a duck or a goose. When obtained from poultry, the mucin containing tissue may also be the egg albumen. When obtained from a mammalian quadruped such as a pig or a cow the mucin containing tissue may be an organ or a part thereof. Thus, the tissue may be a mouth tissue comprising salivary glands, a mucous membrane such as a tongue, an esophagus, a stomach, an intestine, a lung or a mixture thereof. Mucin containing tissue may also be a specific tissue of an animal organ such as the gastric tissue of a pig or a submaxillary gland of a cow.

In another example of mucin isolation from an animal, the mucin containing tissue may originate from a mollusk or a marine mollusk such as a clam or a cockle, wherein the isolated tissue may comprise a suprapedal or submaxillary gland. The mucin containing tissue may also be obtained from a fish. Examples for suitable fish include eel or hagfish. When such fish are used as mucin source, the mucin containing tissue of an eel or hagfish may be its skin. The mucin containing tissue may also be sourced from a cnidarian such as a box jellyfish or a coral.

The mucin containing tissue of animal origin may be selected from the group consisting of a stomach, a submaxillary gland, a suprapedal gland, a lung, an intestine, a mucous membrane, a skin, or a part or a mixture thereof. Preferably, the mucin containing tissue is a stomach.

The tissue may be present in any suitable processable form. The tissue may be used as a whole (for example, if skin or an intestine from a small animal such as a bird or tissue from a plant is used). In such a case, the mucin containing tissue may be everted to facilitate the accessibility of the aqueous solution to the mucin containing tissue. In another example maybe a whole organ is used. If, as an illustrative example, the skin of a whole hagfish is used, the tissue (skin) may be about 30 cm to about 90 cm in the longest dimension. However, the dimensions of the used mucin containing tissue may also be greater. In case the mucin containing tissue has such dimensions it may be further prepared, in that pieces or parts of the mucin containing tissue of plant or animal origin are used. Consequently, the mucin containing tissue may be cut manually or by using a cutting device before it is contacted with the aqueous solution.

It is encompassed herein that a mixture of animal and plant tissue pieces may be used. Likewise it is envisaged that tissues originating from different individual animals may be extracted at the same time. These individual animals may not necessary belong to the same species.

The mucin containing tissue pieces may have any shape, for example an essentially rectangular, square or circular shape. For example, if stomach pieces with an essentially rectangular shape are used, these pieces may have the longest dimension of about 1 cm to about 20 cm, or of about 4 cm to 15 cm. In a preferred embodiment, the mucin containing tissue is a stomach and originates from pork. When preparing the dissected stomach for the mucin isolation the organ may be rinsed briefly, and may be cut to the abovementioned dimensions.

Using (relatively) small tissue pieces may have the advantage that the aqueous solution is able to access the complete mucous surface of the mucin containing tissue which also may contribute to effective mucin isolation.

### Method for isolating mucin

The method of the present invention relates to the isolation of mucin from a mucin containing tissue which is contacted with an aqueous solution over a suitable period of time, which is at least about 10 min, at a suitable temperature, which is in the range of about 0°C to about 42°C, for transferring mucin into the aqueous solution and thus creating a mucin containing solution. As a main aspect of the method of the present invention, the mucin containing solution is then filtered in at least two subsequent filtration steps to remove debris and impurities from the mucin containing solution. Further filtration steps are also envisaged, each using different filters with successively decreasing pore size diameters. In the following steps of the herein described method, the filtered mucin containing solution is further purified by performing a size exclusion chromatography, thereby producing a mucin enriched solution, and diafiltered to reduce the amount of salt in the mucin enriched solution. The diafiltered mucin enriched solution can then be frozen or dry-frozen to store the isolated mucin.

### Contacting the mucin containing tissue with the aqueous solution

In a first step, the mucin containing solution is produced by contacting a mucin containing tissue with an aqueous solution over a suitable period of time, at a suitable temperature as defined somewhere else herein, for transferring the mucin into the aqueous solution. When used for the method of the present invention contacting means in its broadest sense adding the aqueous solution to the mucin containing tissue, or removing the mucin mechanically from the mucin containing tissue and adding the aqueous solution subsequently. Removing the mucin mechanically includes any means or treatment by which the mucin can be removed and collected from the surface of the mucin containing tissue. Preferably, contacting the mucin containing tissue with the aqueous solution comprises manually scraping, brushing, or washing the mucin from the mucin containing tissue into the aqueous solution, or adding the aqueous solution to the mucin containing tissue. However, a contact of the mucin containing tissue with the aqueous solution can also be established without mechanical or manual interfering, simply by adding the solution to the mucin containing tissue over a suitable period of time, at a suitable temperature as described somewhere else herein. Contacting the tissue with the aqueous solution further comprises moistening of the entire mucin containing tissue with the aqueous solution. This way, the solution may access the complete surface of the mucin containing tissue, which facilitates the transfer of mucin into the solution.

The aqueous solution may be added to the collected mucin. Alternatively, the mucin may be removed mechanically and collected into the aqueous solution or the aqueous solution may be added without a mechanical preparation of the mucin containing tissue and left thereon as described herein.

In the case a stomach is used as mucin containing tissue the aqueous solution may be filled directly into the stomach which may be accompanied or aided by shaking or by using a magnetic stir bar which constantly moves the fluid inside the stomach. When preparing the dissected stomach for the mucin isolation, the organ may be rinsed briefly, and may be cut into smaller pieces. In particular, it is envisaged that the mucin containing tissue is optionally cut to an essentially rectangular shape with the longest dimension of about 1 cm to 20 cm, more preferably the longest dimension of about 4 cm to 15 cm, prior to contacting the mucin containing tissue with the aqueous solution. Thus, the pork stomach may be cut into pieces with the abovementioned dimensions before adding the aqueous solution.

Adding the aqueous solution to the mucin containing tissue can be performed by placing the mucin containing tissue in a suitable vessel or bucket and adding the aqueous solution, or aided by a bubble column as describe somewhere else herein. Thus, the process of contacting the mucin containing tissue with the aqueous solution can optionally be performed manually or in a bubble column, as described somewhere else herein. The mucin containing solution which may be produced manually or by using the bubble column is afterwards subjected to the filtration steps, as described herein.

### Reaction conditions for a mucin transfer into the aqueous solution

Mucins are water soluble glycoproteins which enables their transfer into an aqueous solution. Suitable conditions for a facilitated mucin transfer into the aqueous solution may be determined by the temperature, pH or the salt concentration of the solution. Further, the time period over which the mucin containing tissue is contacted with the aqueous solution, may play a role.

### Suitable period of time

In the context of contacting the mucin containing tissue with the aqueous solution, the mucin containing tissue is contacted with the aqueous solution for as short as about 10 min or at least about 30 min. In one example, the mucin containing tissue may be contacted with the aqueous solution over a longer period e.g. for at least about 1h, about 2h, about 3h, about 4h, about 5h, about 6h, about 10h, about 12h or even about one or two days. A suitable period of time for contacting the mucin containing tissue with the aqueous solution is at least about 10 min, at least about 20 min, at least about 30 min, at least about 40 min, at least about 50 min, at least about 60 min, at least about 70 min, at least about 80 min, at least about 90 min, at least about 100 min, at least about 110 min, at least about 120 min. Preferably, a suitable time period is any time period between 30 minutes and 3 hours. These time periods allowing the transfer of the mucin into the aqueous solution may be performed with or without the aid of a bubble column as described herein.

### Suitable temperature

Thus, it is apparent that a transfer of mucin into the aqueous solution is encompassed to include the suitable time period or incubation time. A suitable temperature for the transfer of mucin into the aqueous solution may be influenced by different factors as well. The broadest applicable temperature range is based on the natural requirements of proteins, which denaturize above 42°C and likewise loose structural integrity below 0°C. An efficient transfer using the Brownian motion might be established by higher temperatures in the range of 25°C to 42°C. However, to prevent extensive bacterial growth and thus degradation of the mucin by bacterial processes, the temperature of the aqueous solution may be adjusted. For example, the temperature of the aqueous solution may be in the range of about 2°C to about 6°C, or in the range of about 3°C to 5°C. Further, a bactericidal or bacteriostatic agent may be added to the aqueous solution. Any bactericidal or bacteriostatic agent suitable to avoid or inhibit bacterial growth in an aqueous solution can be used. An illustrative example for a suitable bactericide may be sodium azide or merthiolate. Examples for a suitable bacteriostatic agent include but are not limited to chloramphenicol, macrolides, novobiocin, spectinomycin, sulfonamides and tetracyclines. Said temperatures in the range of about 0°C to about 42°C, in the range of about 2°C to about 6°C, or in the range of about 3°C to 5°C and said bactericidal or bacteriostatic agents may be applied in combination with a bubble column.

### PH of the aqueous solution

A suitable pH is influenced by the pH optimum of digestive enzymes of the stomach which are capable of degrading mucin. The enzyme pepsin, which is found in the gastric juice is active in a range of pH 2 to pH 4 and may be an example for such a mucin degrading enzyme. During contacting the mucin containing tissue with the aqueous solution, impurities such as food residues, gastric fluids or cell lysates may decrease the pH to a level suitable for the activity of such mucin degrading enzymes. Thus, it may be advantageous to adjust the pH of the aqueous solution during the contacting process. In the method of the present invention, the pH may be set in a range of about pH 4 to about pH 9. The pH may, for example, be set to be about 7, or about 8 or to be in the pH range of about 7 to about 8. For pH adjustment and/or the pH maintenance during the contacting process, a buffer solution may be used.

### Suitable amount of aqueous solution

As mentioned above, mucins are water soluble. Therefore, any aqueous solution can be used for the method of the present invention which is suitable for transferring mucin from a mucin containing tissue into the solution. Further, considering osmotic properties it may be advantageous to use a suitable amount of aqueous solution which may facilitates the transfer of mucin into the aqueous solution. A suitable amount of fluid may be crucial for the transfer of mucin into the aqueous solution and thereby for the mucin yield of isolation process. A suitable amount of aqueous solution has been determined by the inventors as ten to fifteen volumes of aqueous solution in relation to one volume of mucin containing tissue. It is within the knowledge of a person with average skill in the art that the respective amount of aqueous fluid needs to be determined in relation to the amount of mucin containing tissue used to extract the mucin. Thus, it might be suitable to weight or measure the volume of the used tissue to prepare an about 1:15 mixture (v/v) or preferably an about 1:10 mixture (v/v) of mucin containing tissue to aqueous solution. It is within the knowledge of a person with average skill in the art that such an amount of aqueous solution has to be prepared before the mucin containing tissue is contacted with the aqueous solution.

The aqueous solution suitable for the present invention may be a saline solution or a saline buffer. An illustrative example for a suitable saline solution is a physiological solution, which may be prepared with salt comprising monovalent cations. An illustrative example for such a salt may be any commonly used salt such as sodium chloride or potassium chloride in which sodium or potassium is the monovalent cation. The saline solution may also be prepared with commonly used buffering salts such as disodium hydrogen phosphate, potassium dihydrogen orthophosphate or monopotassium phosphate. Typically, any aqueous buffer reagent that is able to buffer within a range of about pH 4 to about pH 9 can be used in the present invention. Illustrative examples include, but are not limited to saline solution, phosphate buffer, phosphate buffered saline (PBS) solution, tris-buffered saline (TBS), Veronal-buffer, citrate-phosphate buffer, tris(hydroxymethyl) aminomethane (TRIS), **TE** buffer, 4-(2-hydroxyethyl)-1-piperazineethanesulfonic acid (HEPES) buffer, Ringer's solution or a bicarbonate buffer system. In one illustrative embodiment of the method, the aqueous solution is a sodium phosphate buffered saline solution with about 10 mM sodium phosphate and about 1 M NaCl.In a further illustrative example, the aqueous solution that is contacted with the mucin containing tissue is a sodium phosphate buffered saline solution with at least the concentration of 3x PBS, but preferably about 10 mM sodium phosphate, about 1 M NaCl and about 0,04 % NaN₃..

### Salt concentration of the aqueous solution

As mentioned above, the aqueous solution has a certain salt concentration. The reason for this is that a high salt concentration decreases the Debye screening length, causing attenuated electrostatic interactions of the charged mucin. Thus, a certain salt content in the aqueous solution facilitates the detachment of mucin from the mucin containing tissue or debris and consequently contributes to the liquefaction of the mucus. Further, the salt content of the solution may also influence the quality of the mucin product since impurities such as charged molecules may detach from the mucin structure due to the attenuated electrostatic interactions of the mucin. The concentration of osmotic active compounds of the aqueous solution is reflected in the osmolarity of the solution. Thus, the aqueous solution suitable for the present invention may have an osmolarity of at least about 800 mOsmol/L up to about 2500 mOsmol/L. The aqueous solution of the present invention may also have, for example, an osmolarity of up to about 1000 mOsmol/L, of up to about 1100 mOsmol/L, of up to about 1200 mOsmol/L or of up to about 1300 mOsmol/L, of up to about 1400 mOsmol/L, of up to about 1500 mOsmol/L, of up to about 1600 mOsmol/L, of up to about 1700 mOsmol/L, of up to about 1800 mOsmol/L, of up to about 1900 mOsmol/L, of up to about 2000 mOsmol/L, of up to about 2100 mOsmol/L, of up to about 2200 mOsmol/L, of up to about 2300 mOsmol/L, of up to about 2400 mOsmol/L, of up to about 2500 mOsmol/L. Preferably, the aqueous solution suitable for the present invention has an osmolarity of about 2000 mOsmol/L.

After contacting the mucin containing tissue with the aqueous solution and allowing the transfer of the mucin into the aqueous solution, the liquid phase containing the mucin (herein termed mucin containing solution) is separated from the remaining solid tissue. In the simplified method of the present invention such a separation is established by filtration without the need for centrifugation. This can be established, as mentioned above, through a high salt concentration of the aqueous solution with an osmolarity of at least about 800 mOsmol/L. The filtering process comprises at least two filtration steps, more preferably three filtration steps. Further filtration steps, such as four of five filtration steps are also envisaged by the method of the present invention.

### Filtration steps

Isolating mucin from a mucin containing solution using the method of the present inventioncomprises subjecting the mucin containing solution to at least two subsequent filtration steps, wherein the pore size diameter of the first filter used in the first step and the filter pore size diameter of the second filter used in the second step are different, and wherein the second filter used in the second step has a smaller pore size diameter than the first filter used in the first step, thereby producing a filtered mucin containing solution.

Each of the at least two filtration steps is repeated at least once, meaning the mucin solution may be filtered several times through a filter having the same pore size diameter or a pore size in the range given herein for the respective filtration step before the next subsequent filtration step is performed. The subsequent filtration step uses a filter with a smaller pore size diameter than the previous filtration step. Thus, the mucin containing solution is filtered through filters with successively decreasing pore sizes. The first filtration step uses a first filter with a pore size diameter in the range of about 3 mm to about 0.5 mm, meaning of about 3 mm, about 2.5 mm, about 2 mm, about 1.5 mm, about 1 mm, about 0.5 mm. While the pore size employed in this first filtration step is not limited to this range, in one illustrative example, the pore size diameter of the first filtration step is about 1 mm. A mesh may be used as filter for the first filtration step. The mesh used herein may be made of any one of nitrocellulose, mixed cellulose ester (MCE), glass, glass fiber, glass foam, plastic, polypropylene (PP), polyether sulfone (PES), polytetrafluoroethylene (PTFE), polyvinylidene fluoride (PVDF), nylon, tissue, felt, wool, fleece or metal. In one illustrative example, the mesh is preferably a stainless steel mesh. Even more preferably the first filtration step uses a steel mesh as first filter, with the pore size diameters indicated herein for the first filtration step.

The second filtration step uses a second filter with a pore size diameter in the range of about 600 µm to about 60 µm, meaning of about 600 µm, about 500 µm, about 400 µm, about 300 µm, about 200 µm, about 100 µm, about 90 µm, about 80 µm, about 70 µm, about 60 µm. While the pore size employed in this first filtration step is not limited to this range, in one illustrative example, the pore size diameter of the second filtrating step is about 100 µm. A filter may be used for the second filtration step, and the filter may be a paper filter, a nitrocellulose filter, a mixed cellulose ester (MCE) filter, a glass filter, a glass fiber filter, a glass foam filter, a syringe filter, a filter sleeve, a sterile filter unit, a plastic filter, a polypropylene (PP) filter, a polyether sulfone (PES) filter, a polytetrafluoroethylene (PTFE) filter, a polyvinylidene fluoride (PVDF) filter, a nylon filter, a tissue filter, a felt filter, a wool filter, or a fleece filter, but preferably a stainless steel mesh. It has been found by the inventors that a metal mesh or filter produces a better flow rate of the mucin containing solution than a nylon filter or membrane does **(****Figure 8****).** Thus, the second filtration step may preferably use as second filter a stainless steel mesh or filter with the pore size diameters indicated herein for the second filtration step.

The method of the present invention may comprise a third filtration step. The third filtration step may use a third filter with a pore size diameter in the range of about 60 µm to about 10 µm, meaning about 60 µm, about 50 µm, about 40 µm, about 30 µm, about 20 µm, about 10 µm. Preferably, the third filtration step uses a pore size diameter of about 40 µm. Even more preferably the third filtration step uses a third filter which is a paper or tissue filter with said pore size diameter. An example for further suitable filters also encompassed by the method of the present invention may be a paper filter, a membrane filter, a glass fiber filter, a syringe filter, a filter sleeve or a sterile filter unit. In a purely illustrative example, a commercially available tea or coffee filter may also be used.

In another embodiment of the method of the present invention the mucin containing solution may be filtered through filters with successively decreasing pore sizes, wherein each filter is used only once and wherein more than four filters with decreasing pore size diameters are used consecutively.

The filtration process of one or more steps is used to separate impurities or debris from the mucin containing solution and produces a filtered mucin containing solution and each of the filtration steps may be repeated at least once before the next subsequent filtration step is performed. It is apparent from the method described herein that no centrifugation step is used. A subsequent sequence of centrifugation steps, as described for the method of Schömig et al., 2016, requires several preparation steps and prolongs the complete isolation process (see also **Figure 4****).**

However, it is further encompassed that any separation means suitable for restraining the solid tissue or impurities from the liquid phase may be used in addition to the method described herein. For example, the tissue may also be separated by decanting, straining, sieving, centrifuged or separated by the use of a disc separator. The filtered mucin containing solution which may thus be subjected to further separation or purification. Thereby, the mucin is purified from impurities such as cellular components. In one example, the mucin containing solution may be contacted with a chemical for a certain period of time to ensure a binding or denaturation of the impurities. In a following step, the chemical containing mucin solution may be filtered so that impurities remain in the filter. For this purpose, any chemical suitable for denaturizing proteins in a physiological solution can be used. Examples for such suitable chemicals are urea or guanidine hydrochloride. In one example, the mucin containing solution may be contacted with about 4 M, or about 5 M, or about 6 M, or about 7 M, or about 8 M urea for about 10 min to about 24 h, for example for about 3 h to bind cellular impurities. Thus, the impurities may precipitate allowing their removal by centrifugation leaving the mucin in the supernatant. In an alternative example for purifying mucin from cellular impurities, the mucin containing solution may be further subjected to filtration or ultracentrifugation leaving the purified mucin in the supernatant.

### Size exclusion chromatography

The mucin containing solution may be subjected to a size exclusion chromatography (SEC) after filtration to remove lipids, macromolecules such as polymers or proteins or previously added compounds such as bactericidal or bacteriostatic agents. The SEC may be carried out by using a saline solution or saline buffer. The salt content in the solution or buffer decreases the electrostatic interactions of the mucin molecule, thereby improving the purification and the quality of the mucin. Any saline solution or saline buffer with high salt content can be used for the SEC step of the present invention. As an illustrative example, the filtered mucin containing solution is subjected to a SEC with a saline buffer, wherein the saline buffer is preferably 1x, 2x or 3x PBS.

### Diafiltration

After the SEC, the mucin containing solution may be subjected to a diafiltration to concentrate and further purify the mucin. Also, the diafiltration may be performed with a saline solution or saline buffer. For example, the salt concentration of the saline solution or saline buffer used for the diafiltration may have a salt concentration in the range of about 200 mM to about 5 M, or in the range of about 1.5 M to about 2.5 M. In an illustrative example a 10 mM sodium phosphate solution with about 1 M NaCl is used for the diafiltration. The present method may also comprise a desalination of the diafiltrated mucin containing solution using distilled water or ultrapure water. By desalinating the mucin containing solution, quality fluctuations may be avoided since the electrostatic interactions of the mucins of the present invention are reduced to an equally low conductivity level in every batch. For example, the salt concentration of the diafiltrated mucin containing solution is reduced to a conductivity of at least <50 µS/cm, more preferably <25 µS/cm, most preferably <10µS/cm. Finally, the purified mucin solution may optionally be frozen at -80°C for long-term storage. Alternatively, the mucin may be dried for long-term storage. In this context, any drying procedure available to a person skilled in the art may be used. For example, the purified mucin may be freeze-dried, lyophilized or spray-dried.

### In Bubble column

The present invention includes, in one embodiment, the use of a bubble column for contacting a mucin containing tissue with an aqueous solution and further isolating said mucin. The bubble column suitable for the present invention may comprise components depicted in **Figure 3****,** i.e. a hollow chamber (1), which may be sealed to the outside, a gas inlet (2), a non-return valve (3), a gas outlet (4), an external cooling system (5) and a control system for pH and a control system for the conductivity of the liquid. The gas inlet (2) of the bubble column may comprise several nozzles on the bubble column bottom. The gas outlet (4) of the bubble column may be mounted on the top or the upper side of the hollow chamber (1) of the bubble column. However, any bubble column of similar properties aerating a hollow chamber capable of containing an aqueous solution and thus mixing the aqueous solution with the mucin containing tissue can be used. Such a bubble column consequently facilitates the transfer of the mucin into the solution.

The bubble column can be made from any suitable material. The bubble column may be of poly methyl methacrylate (PMMA), stainless steel or glass, to mention only a few suitable materials. Likewise, the bubble column may have any suitable dimensions, depending on the intended capacity, for example. As an illustrative example, the bubble column may have an inner diameter of at least about 100 mm to about 250 mm and a height of at least about 150 mm to about 750 mm. Thus, the bubble column may have a volume of about 1 liter to about 37 liters. Consequently, in one illustrative example the bubble column may have at least an inner diameter of about 190 mm and at least a height of about 500 mm resulting in a volume of about 14 liters.

In one embodiment, the bubble column is optionally equipped with a filter and a return system to prevent foam from leaking into the gas outlet and to return the liquid to the system.

It is within the knowledge of the person of average skill in the art to choose an experimental setup, considering the size of the bubble column, the gas flow and the flow regime, that ensures that mucin can be isolated from larger tissues as well as from smaller tissue pieces thereby maintaining the same effectiveness and mucin yields.

In the method of the present invention, the bubble column is used to contact the mucin containing tissue with the aqueous solution. This may be advantageous because the mucin containing tissue can simply be cut (if necessary) to suitable dimensions as mentioned somewhere else herein. The tissue and the aqueous solution can be added together into the bubble column. Thereby, both can be carried out in any sequence, for example the mucin containing tissue and the aqueous solution can be added to the bubble column. This means the aqueous solution can be added into the bubble column and the mucin containing tissue can be added subsequently or in reverse order.

In a preferred embodiment, the mucin containing tissue is a stomach and originates from pork. When preparing the dissected stomach for the mucin isolation the organ may be rinsed briefly, cut into pieces and added into the bubble column. By using the bubble column for contacting the mucin containing tissue with the aqueous solution the manual removal of mucins from the tissue is rendered dispensable. However, a manual removal of the mucin may be performed after rinsing the mucin containing tissue and before cutting the tissue into pieces which are then placed in the bubble column. Thus, it is also encompassed by the method of the present invention that the mucin may be mechanically removed in a first step, and the remaining tissue may be added into the bubble column to efficiently remove residual mucins.

An efficient transfer of the mucin into the aqueous solution is enabled by the constant air or gas flow into the hollow chamber filled with the aqueous solution, causing a bubble driven circulation of the fluid and thus gently removing the mucin from the surface of the mucin containing tissue. For the aeration of the aqueous solution any gas may be used, wherein the gas may be preferably air or an inert gas or a mixture thereof. An example for an inert gas may be nitrogen. However, while economically not preferred, also a noble gas such as helium, neon, argon, krypton, xenon, or radon may be used as inert gas. The gas flow generates bubbles in the aqueous solution, which cause shear and flow forces on the mucous surface of the mucin containing tissue. Thus, the bubble driven circulation may contribute to transferring the mucin into the aqueous solution.

As mentioned before, the bubble column may contain the aqueous solution such as a saline solution or a saline buffer as described somewhere else herein to isolate mucin from a mucin containing tissue. In another embodiment the aqueous solution may be 3x PBS having an osmolarity of about 840 to 945 mOsmol/L, or sodium phosphate buffered saline solution with about 10 mM sodium phosphate with about 1 M NaCl and about 0,04 % NaN₃ having an osmolarity of about 2108.6 mOsmol/L. The aqueous solution therefore has an osmolarity of at least about 800 mOsmol/L up to about 2500 mOsmol/L. The amount of aqueous solution which needs to be added to the mucin containing tissue is preferably about 10 to about 15 volumes aqueous solution to 1 volume of mucin containing tissue. However, to operate the bubble column 2 volumes of aqueous solution may be combined with 1 volume of mucin containing tissue. This means that the size of the bubble column has to be adapted to the volumes of mucin containing tissue and aqueous solution used, prior to the isolation process.

After the hollow chamber of the bubble column is filled with the aqueous solution/ mucin containing tissue mixture, the air or gas flow and the cooling system are switched on and the mixture incubates for a suitable amount of time. A suitable time period is, as mentioned already, preferably any time period between 30 minutes and 3 hours.

It is encompassed by the present invention that the mucin containing solution resulting from the incubation in the bubble column is further processed as described herein. This means, the mucin containing solution is, in a preferred embodiment, subjected to the filtration process, the size exclusion chromatography and diafiltration as described herein. However, in another embodiment not part of the invention, the mucin containing solution may be centrifuged after the described incubation time in the bubble column, to purify the mucin containing solution from the remaining tissue. Such a centrifugation step may be an ultracentrifugation at about 45000rpm (Ti-45)/150000 x g for about 1h. A thereby created supernatant is consecutively subjected to size exclusion chromatography and diafiltration as described herein.

### The obtainable mucin

Described herein is a mucin which can be obtained by the method described herein. Such a mucin has lubricious properties, which are comparable or superior to the properties of mucin isolated manually after the protocol of Schömig et al., *supra.* Thus, the present invention provides a mucin which may be structurally undamaged or less damaged and therefore more lubricious than the commercially available mucin products as shown by **Figure 6****.** The structural integrity of the mucin molecule isolated according to the method of the present invention may be detectable by using antibodies in an enzyme-linked immunosorbent assay (ELISA; **Figure 5****).** Therefore, the mucin of the present invention may be more suitable for the use in a (molecular) biological or medical application and/or for use in a medical product or compound. For example, the mucin of the present invention may be a more suitable biological lubricant for the use in a medical product or more suitable for the use on a medical device. The mucin obtained by the method of the present invention may also be more suitable for the use as an epidermal barrier against invading pathogens.

The above being said, the present invention provides an easy and time efficient method for the isolation of mucin, and achieves a higher yield as compared to the manual mucin isolation of Schömig et al., *supra.* Thus, when pig stomachs are used, the mucin yield is increased from about 65 mg per stomach to about 200-300 mg per stomach by the present invention.

The invention will be further illustrated by the following non-limiting Experimental Examples.

### EXPERIMENTAL EXAMPLES

### Example 1: PGM isolation by using an aerated bubble column (not part of the invention)

### Materials:

### Body parts of the bubble column (custom-made from PMMA)

- Cylinder (*H* = 500 mm, *d*ᵢ = 190 mm, *d*ₐ = 200 mm) with two flanges (containing 6 regularly orientated M5 threads each) to attach bottom and top lid
- Bottom plate containing 5 regularly orientated holes (*d* = 3 mm) for gas supply
- Bottom lid with a central G1/4 thread for gas supply
- Top lid with central G1/4 thread as a gas exit
- Lid for gas overflow filter
- Overflow connector with thread for 0.5 L flask and overflow lid (both made from stainless steel)

### Labware for assembling the bubble column

- 0.5 L flask for overflow filter
- Pneumatic tubings for gas supply (*d*ᵢ = 6 mm, *d*ₐ = 8 mm)
- Manometer (0 - 10 bar)
- Gas valve
- O-rings
- Steel grid (1 cm grid size)
- M5 bolts
- Pneumatic connector (with thread)
- Pneumatic clutches
- Non-return valve
- 10 L beakers

### Labware for Mucin extraction:

- beakers, 1 x 2 L (plastic), several 5 L
- garbage bags for the stomachs (~50 L)
- pH meter
- Pipetboy + serological pipettes, 50 ml
- 1x Schott flask 2 L
- bottletop dispenser
- centrifuge tubes, 50 mL (~100 per 20 stomachs)

### Labware for Centrifugation and SEC:

- Beckmann Optima LE70 ultra centrifuge
- 6x 70 mL Beckmann centrifuge tubes for ultra-centrifugation
- GE Healthcare ÄKTA purifier system
- XK50/100 column packed with Sepharose 6FF
- glass cylinder, 250 mL
- Schott flasks, 250 mL, 1 L, 2 L, several 5 L
- (reuse) Falcon centrifuge tubes, 50 mL (8 per SEC run)

### Labware for Crossflow filtration:

- Schott flasks, 1x 5 L, 1x 2 L
- beakers 2x 0.5 L, 5 L
- conductometer
- graduated cylinder, 250 mL
- Eppendorf tubes, 5 mL (~20 per run)
- centrifugation tubes, 50 mL (~3 per run)
- dispenser pipette + 50 mL combitip

### Consumables and chemicals

- 2-propanol
- Milli-Q water
- Paper towels
- 1x PBS
- 3x PBS (contains 18.36 mM Na₂HPO₄, 11.64 mM NaH₂PO₄ and 510 mM NaCl, pH = 8)

### Assembly of the bubble column

All parts of the bubble column were cleaned with 2-propanol. Afterwards, an O-ring was placed in the bottom flange of the main cylinder before the bottom plate was placed onto the flange. A second O-ring was placed in the bottom lid, before the bottom lid was placed onto the bottom plate. Six M5 bolts were used to screw the bottom lid, bottom plate and main cylinder together. Then, an O-ring was placed in the top flange of the main cylinder. Afterwards, a pneumatic connector was installed to the central thread in the bottom lid. Pneumatic tubing with a length of about 20 cm connected the pneumatic connector with a non-return valve. The non-return valve was also connected to a closed gas valve via another pneumatic tubing of 20 cm length. A pneumatic tubing was also used to connect the gas valve to a manometer and to a pneumatic clutch, which was later used as a coupling piece for the gas supply. Another pneumatic connector was installed on the central thread in the top lid, before it was connected to an overflow connector placed on a 0.5 L flask via a pneumatic tubing. Upon the overflow connector, a piece of paper towel was placed before the overflow lid was fixed with four M5 bolts.

### Operation of the bubble column

The bubble column was operated at 4°C. Thus, the assembled bubble column was placed in a cold room and then connected to compressed air supply (oil free, 10 bar) via the pneumatic clutch. Four stomachs of freshly slaughtered pigs (about 2 liters porcine stomach) were cut into 6 pieces and put into the bubble column. Then, 4 liters of pre-cooled (4°C) 3x PBS was added to the porcine stomachs in the bubble column (i.e. one volume of mucin containing tissue is contacted with twice the volume of the saline buffer in the bubble column). After the top lid of the bubble column was fixed with six M5 bolts and connected to the overflow flask via pneumatic tubing, the gas flow was started carefully. The gas pressure was slowly adjusted to 3 bar using the manometer. Stomach pieces and buffer were mixed for about 3 h before the gas supply was stopped. The gas supply was dismounted behind the non-return valve to avoid fluid from leaking. The top lid was dismounted and the contents of the bubble column comprising the porcine stomachs and the mucin containing solution was decanted into a 10 L beaker using a grid. The mucin containing solution was then subjected to ultracentrifugation. If necessary, the mucin containing solution can be stored at -80°C until further use. The bubble column was then disassembled and cleaned with Milli-Q water and 2-propanol.

### Ultracentrifugation of the mucin containing solution

In a first step, 70 ml centrifuge tubes (Beckmann) were filled up to 90% with the decanted mucin containing solution. After taring, the closed centrifuge tubes were carefully inserted into a Ti-45 rotor, and the rotor was inserted into the ultracentrifuge without scratching the coding disk at the bottom of the rotor. The mucin containing solution was ultracentrifuged at 4°C and 45000 rpm (Ti-45)/150000 x g for about 1h. Afterwards, the supernatant of all centrifuged tubes was decanted into one 2I Schott flask. All tubes and also the 2I Schott flask were kept at 4°C when not in direct use. The collected and pooled supernatant of all runs can be divided into 50 ml Falcon tubes and stored at -80°C for further use. The ultracentrifuge was cleaned manually. An alternative for this step is a filtration.

### SEC of the mucin containing solution

First, the supernatant collected after the ultracentrifugation was poured through a paper filter (commercially available tea filter) into a 250 ml glass cylinder and stored at 4°C. Then, an ÄKTA purifier system was prepared for SEC by purging the sample inlet to be used with 50 ml 1x PBS. 50 ml Falcon tubes were placed into the autosampler and the fractionation volume was adjusted to 44 ml per falcon tube. A 5I bottle of 1x PBS and the 250 ml glass cylinder containing the mucin containing solution were connected with the ÄKTA purifier system using inlet tubes. After the waste bottle was emptied the purification was started manually. After about 90 min the automatically collected samples were harvested. At this point the collected samples can be also stored at -80°C for further use. The ÄKTA purifier system was cleaned after the run was completed.

### Crossflow-Filtration

The samples of 3 ÄKTA runs were thawed overnight at 4°C (alternatively in a water bath at room temperature on the day of crossflow filtration). The mucin containing solution was filled into the reservoir of the clean crossflow filtration device. Then, the osmolarity of the solution was adjusted by adding sodium chloride (NaCl) until a NaCl concentration of 2M is reached. Until all NaCl was dissolved, the pump was operated at low speed (max. 200 rpm). Afterwards, the pump was operated at 400 rpm and about 10 psi retentate pressure until the mucin solution was concentrated to a volume of about 300 ml. By using the Rinsing/Diafiltration mode, the mucin solution was dialyzed against ultrapure water until a conductivity of ≤ 10 µS/cm was reached (400 rpm and ~ 10 psi retentate pressure). Then, the mucin solution was concentrated to a volume of about 70 ml to 90 ml. The concentrated mucin solution was aliquoted in labelled 5 ml Eppendorf tubes (4.5 ml per aliquot) and stored at -80°C.

### Lyophilization

The purified mucin solution was freeze dried. For this, holes were drilled into the lids of the still frozen 5ml Eppendorf tubes. Then, these prepared frozen Eppendorf tubes were put into the vacuum chamber. After 2 to 3 days the mucin was freeze-dried and ready to be harvested.

After lyophilization, a mucin yield of about 200 mg per porcine stomach was determined. This is a significant improvement compared to the manual mucin isolation according to Schömig et al. which yields only 65 mg per stomach. Thus, the present invention provides a significant improvement for mucin isolation since the method described herein provides for a time efficient, effective and scalable isolation of mucin while at the same time yields about three times more mucin than the prior art with constant quality (as shown in the following Examples 2 and 3).

### Example 2: Experiment showing that the mucin isolated according to the present invention is detectable by mucin-specific antibodies in contrast to commercially available mucin

To test the mucin (MUC5AC) content in an aqueous solution, indirect enzyme linked immunosorbent assays (ELISA) were performed with two commercially available mucin variants and the manually purified mucin. Therefore, a solution containing 0.00001-0.1 % (v/w) mucin was pipetted into wells of a 96 well cell culture plate and incubated for 1 h to allow the mucin to adsorb to the surface of the wells. Afterwards, all samples were removed, and the wells were filled with a blocking buffer (5 % milk powder dissolved in PBS-Tween, i.e. PBS containing 1 mg/mL Tween 20, Carl Roth, pH = 7.4) and incubated at 4 °C overnight.

On the next day, the blocked wells were rinsed with PBS-Tween for three times. Afterwards, the samples were incubated with a primary antibody (200 µL per well) for 60 min while being placed on a shaker (Promax 1020, Heidolph Instruments GmbH & Co. KG, Schwabach, Germany). For this step, specific antibodies for MUC5AC detection (ABIN966608, antibodies-online GmbH, Aachen, Germany) were used. The recognition sequence of these mucin antibodies is located in the non-glycosylated C-terminus of the mucin molecule. A damage or complete absence of this C-terminus or even damage in the glycosylation pattern may cause a conformational change of the molecule, which again may result in inaccessibility to the antibodies. Thus, the binding of antibodies to MUC5AC isolated according to the present invention is a qualitative feature for mucin integrity and thus for native mucin.

The mucin antibodies were diluted 1:400 in blocking buffer. After incubating, the samples were rinsed again with PBS-Tween. A second antibody staining was then performed using horse radish peroxidase (HRP) conjugated goat anti mouse (murine) IgG antibodies (ABIN237501, antibodies-online GmbH). These secondary antibodies were diluted 1:5000 in blocking buffer. Incubation was allowed to take place for 120 min on a shaker at room temperature (RT). Afterwards, the samples were washed in pure PBS (since Tween tends to interfere with the solutions used for following steps). After washing the samples, 100 µL of QuantaRed Working Solution consisting of 50 parts QuantaRed Enhancer Solution, 50 parts QuantaRed Stable Peroxide and one part of QuantaRed ADHP Concentrate (QuantaRed Enhanced Chemifluorescent HRP Substrate Kit 15159, Thermo Fisher Scientific, Waltham, Massachusetts, USA) were added to each well. Since the Working Solution is light sensitive, direct light contact was avoided.

After 30 min of incubation at RT, the peroxidase activity was stopped by adding 20 µL of QuantaRed Stop Solution to each well. The fluorescence signal of the converted substrate was measured with a multi-label plate reader (Viktor3, PerkinElmer, Inc., Massachusetts, USA). Fluorescence was measured at a wavelength of 570 nm using a data acquisition time of 0.1 s.

The inventors could determine that the two commercial mucin variants could hardly be detected with the specific anti-PGM antibody (Figure 5a and b). In comparison, the manually purified mucin could be detected by a colour reaction visible to the naked eye, even at a mucin concentration of 10⁻⁷ (w/v). When quantifying the resulting fluorescence signals, it was found that the fluorescence signal of manually purified mucin (at a mucin concentration of 10⁻⁷ (w/v)) differs from signals determined for the two commercial mucins by ~2 orders of magnitude. Because the antibody MUC5AC mediates a specific binding to the non-glycosylated C-terminus of the mucin molecule, the inventors assume that either this part is damaged, or by a conformational change of the mucin no longer accessible. Therefore the commercial mucin is not suitable for molecular biological applications, e.g. as a standard for the analysis of clinical mucus samples, while the manually purified mucin is suitable.

### Example 3: Friction tests showing the lubrication properties of mucin isolated and purified according to the present invention in comparison to commercial available mucin

For the friction different mucins were compared. The result were conducted with commercially available PGM (Sigma Type II and Type III), mucin purified according to Schömig et al., and two mucin variants, which have been purified by using the bubble column and the manual filtration process of the present invention, respectively. The manual filtration process which was used to generate the mucin shown in **Figure 6** is described in the following.

### Procedure:

Porcine gastric mucin MUC5AC was purified from porcine gastric mucus. 30 porcine stomachs were obtained from a local slaughter house on the day of processing. Mucus was obtained from gently rinsed pig stomachs by manual scraping of the inner surface of the gastric tissue. The collected mucus (~ 1 L) was diluted 10-fold in 10 mM sodium phosphate buffer (pH 7.0) containing 1 M NaCl and 0.04 % (w/v) NaN₃, and stirred at 4 °C overnight. The homogenized mucus (~10 L) was then filtered through a mesh made from stainless steel with a pore size diameter of 1 mm twice. For storage, the mucus was stored at -80 °C in aliquots of 45 ml. For further processing, 5 aliquots were thawed and the mucus was subjected to another filtration step using a membrane with a pore size diameter of 100 µm. Afterward, the filtrate was poured through a paper filter (commercially available tea filter) into a 250 ml glass cylinder. Subsequently, the mucins were separated from other macromolecules by size exclusion chromatography using an ÄKTA purifier system (GE Healthcare) and an XK50/100 column packed with Sepharose 6FF, which has been equilibrated in 10 mM sodium phosphate buffer (pH 7.0) containing 1 M NaCl. In total, 352 ml of eluate were collected. The collected fractions of 2 ÄKTA runs (= 2x 352 mL) were pooled and dialyzed against ultrapure water until a conductivity of ~10 µS/cm was reached using a crossflow filtration setup. The mucin solution was concentrated to a volume of ~100 ml, and this concentrate was then lyophilized and stored at -80 °C.

### Yield:

According to this procedure, 5 purification batches of mucin have been purified. The mucin yield per stomach determined from these five batches is ~231 mg. The mucin yield per stomach obtained by Schömig et al. was ~65 mg, meaning the method of the present invention to isolate mucin is not only easier and more efficient to carry out but also provides a yield that is about 4 times higher than the method of Schömig et al.

### Friction measurements:

For friction measurements, a commercial shear rheometer (MCR 302, Anton Paar, Graz, Austria) was equipped with a tribology unit (T-PTD 200, Anton Paar). The measurements were performed in a ball-on-cylinder geometry. As friction partners in the tribology setup, three PDMS cylinders (as described earlier) and steel spheres with a diameter of 12.7 mm (1.4301, *S*_{q} < 0.2 mm; Kugel Pompel, Vienna, Austria) were used. Measurements were performed at a constant normal load of *F*_{N} = 6 N. This normal load was chosen such that friction in the boundary, mixed, and hydrodynamic regimes could be probed within the accessible speed range. According to the Hertzian contact theory using the Young's moduli and Poisson's ratios of steel (*Eₛₜₑₑₗ =* 210 GPa, *vₛₜₑₑₗ* ≈ 0.30) and PDMS (*E_{PDMS} =* 2 MPa, *v_{PDMS}* ≈ 0.49), this normal load results in an average contact pressure *p*₀ of ~0.31 MPa.

The speed-dependent friction behavior was evaluated by performing a logarithmic speed ramp from ~700 to 0.001 mm s⁻¹. The friction coefficient was measured at 48 distinct speed levels for 10 s each. Before the first measuring point, the system was allowed to stabilize at the highest rotational speed for 30 s. For each measurement, 600 µL of 0.1 % (w/v) solutions of the respective mucins (dissolved in 20 mM HEPES pH 7.0) were used as lubricants.

Intact mucin, including the manually purified PGM, is an excellent lubricant. The Lubricity of commercially available mucins on the other hand has been almost completely lost **(****Figure 6****).** Manually purified mucin proved to be a highly efficient lubricant - regardless of which manual process the inventors use for purification of the mucin. Already a small amount of 0.1 % (w/v) of manually purified PGM was able to reduce the coefficient of friction by about two decades compared to pure water - and even at extremely low relative speeds, where interfacial friction typically occurs (and which are physiologically relevant; **Figure 6****).** The achievable coefficients of friction were in the range of µ ~0.01 and thus far below the capability of aqueous-, synthetic-, or oil-based lubricants. If instead a solution of commercial mucin is used as lubricant, a shift in the range of interfacial friction towards somewhat slower friction speeds could be observed. Nevertheless, the coefficient of friction finally reached the same value which could be determined for water (~1; **Figure 6****).**

### Example 4: Adsorption onto the silicone

To acquire meaningful research results and for the medical purposes, commercial mucins are unsuitable. In several studies it has been shown that these mucins are structurally damaged - probably due to the aggressive purification process and have lost some of their characteristic properties. For example, the property of mucins to act as a barrier with selective permeability due to the pronounced negative charge of the mucin molecule. Two studies recently showed that commercially available porcine gastric mucin (PGM) do not have a pronounced charge affinity to positively charged particles, as it is usual for functional PGM, but have an increased affinity for negatively charged particles. This indicates that the protein glycosylation is damaged and the zwitterionic protein backbone is exposed. However, not only the glycans anchored to the protein backbone appear to be damaged. Experimental data suggests that the hydrophobic parts of the commercial mucins have become inaccessible either by splitting them off or by changing the protein conformation.

The adsorption of mucin molecules via their hydrophobic temrini onto hydrophobic PDMS surfaces was quantified using QCM-D and the frequency shifts were recorded using a commercial software (qCell T Q2 1.9 and qGraph Viewer 1.8; both 3t-analytik). First, gold coated quartz sensors (3T-analytik) were spin-coated with a thin layer of PDMS as follows: PDMS pre-polymer and cross-linker (Sylgard 184, DowCorning, MI, USA) were mixed in a ratio of 10:1. Then, 10 µL of this PDMS premix were added to 1 mL of n-hexane, and 100 µL of this PDMS/n-hexane solution were pipetted onto the middle of the gold-coated chip surface while rotating the chip on a spin-coater (WS-650MZ-23NPPB, Laurell, North Wales, PA, USA). These rotations were conducted first at 1500 rpm for 20 s and then at 3000 rpm for 60 s. After drying the samples at room temperature overnight, measurements were performed at 30 °C. First, the quartz crystals were rinsed with 20 mM HEPES (pH 7.0) at a flow rate of 100 µL min⁻¹ until a stable frequency baseline signal was obtained. For the adsorption of mucin molecules, a 0.01 % (w/v) mucin solution (generated in 20 mM HEPES, pH 7.0) was injected into the test chamber at a flow rate of 100 µL min⁻¹ and the resulting frequency shift was monitored for 45 min.

These QCM (quartz crystal microbalance) measurements allow to determine the adsorption efficiency of molecules. Thereby, a change in the resonance frequency of a vibrating quartz sensor modulated by the adsorption of molecules onto the quartz surface is measured over a certain period of time. The adsorbed mass can be determined by a simple mathematical relationship and can be determined from the frequency difference, where a large frequency difference corresponds to a large mass of adsorbed molecules. In the laboratory the inventors have compared the adsorption of PGM that was manually purified according to Schömig et al. with that of the two most common commercially available mucin variants (Sigma PGM Type II and Type III; where it was assumed that the commercial mucin variants have the same molecular weight like manually purified, intact PGM) onto a hydrophobic silicone surface. In the process, the inventors were able to determine that the purified mucin adsorbs significantly more on the silicone surface than the two commercial variants **(****Figure 7****).** Efficient adsorption of mucins on surfaces is essential for Surface processes (e.g. lubrication of surfaces).

### Example 5: Method improvement

To reduce the various limitations of the manual purification process **(****Figure 2 A****)** the inventors have modified the purification protocol **(****Figure 2 B****).** These changes are intended to be significantly more time-efficient, to guarantee a higher mucin yield and reproducibility through a simplification of the process (e.g. saving of expensive centrifuges). The aim of the process optimization is to produce a consistently high quality mucin in high yields that can be used for commercial distribution, for research and medical technology applications. At the beginning of the purification process is the harvest of the mucus. This can be done manually. In this case it is necessary to sufficiently (~1:10 to 1:15) mix the harvested mucus with 3x PBS. This dilution step ensures that the pores of the filter membranes are not clogged with viscous raw mucus. By using a higher concentrated buffer (3x PBS instead of 1x PBS) the effect of Debye screening in the mucus solution is increased (=lower Debye screening length), i.e. electrostatic interactions between charged motifs of mucin molecules and other oppositely charged molecules are attenuated. Thus, potential contaminations of the mucin through electrostatic adhesion of impurities are reduced. At the same time electrostatic interactions between mucin molecules are reduced and thus mucin agglomerates are dissolved (=liquidation of the Mucus). The inventors could determine that the salinity of the aqueous solution influences the amount of impurities in the final product. These results could be achieved with a 3xPBS as homogenization buffer. Furthermore, a higher concentrated buffer prevented a drop of the pH-value into acidity, and thereby an undesirable re-gelation of the mucus.

### Example 6: Determining flow rates through nylon membranes and meshes made from stainless steel

Mucus from 30 pig stomachs was harvested by manually scraping the inner surface of the stomachs. A total amount of ~1 L was collected and diluted 10-fold in 10 mM sodium phosphate buffer (pH 7.0) containing 1 M NaCl and 0.04 % (w/v) NaN₃ (having an osmolarity of about 2108.6 mOsmol/L), which yielded ~10 L of mucus. For homogenization, the diluted mucus was stirred at 4 °C overnight.

To remove crude contaminants such as food debris and pieces of gastric tissue, in a first filtration step, the homogenized mucus was filtered through mesh made from stainless steel with a pore size diameter of 1 mm twice. For a second filtration step, meshes with a pore size diameter of ~100 µm were used. Two different materials were tested, namely nylon and stainless steel. The flow of mucus through these membranes was quantified as follows: In each experiment, a volume of 50 mL homogenized mucus (after the first filtration step) was filtered either through a nylon membrane or a steel mesh into a measuring cylinder. The effective filtration area was 3.80 cm² and the total volume was applied onto the filtration membrane at once. Before each experiment, the membranes were soaked in tap water. The time required to filter the entire volume through the respective membrane was determined (no external forces were applied to accelerate the filtration). After each experiment the membranes were rinsed with tap water. The experiments were performed in triplicates.

### Results:

The entire volume of 50 mL could be filtered through the steel meshes successfully. The time required was 28 ± 2 s. For nylon membranes, the mucus flow stopped at some point and the remaining mucus could not seep through the pores of the membranes without additional stirring. A volume of 33 ± 3 mL was filtered through the nylon membrane until the mucus flow ceased. The time required for the filtration of this volume was 56 ± 4 s.

To be able to compare the data obtained for the different membrane types, the experiments were repeated. However, this time, the time required to obtain a filtrate volume of 20 mL was determined. No further modifications were made to the experimental procedure.

When a steel mesh was used for filtration, the time required to obtain 20 mL of filtrate was 8 ± 1 s. To obtain the same amount of filtrate using nylon membranes a time of 24 ± 3 s was required **(****Figure 8****).**

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied herein disclosed may be resorted to by those skilled in the art, and that such modifications and variations are considered to be within the scope of this invention as set out in the claims.

When used herein "consisting of" excludes any element, step or ingredient not specified in the claim element. When used herein, "consisting essentially of" does not exclude materials or steps that do not materially affect the basic and novel characteristics of the claim.

### REFERENCES

- Lieleg, O., Lieleg, C., Bloom, J., Buck, C. B. & Ribbeck, K. Mucin biopolymers as broad-spectrum antiviral agents. Biomacromolecules 13,1724-1732 (2012).
- Käsdorf, B. T. et al. Mucin-Inspired Lubrication on Hydrophobie Surfaces. Biomacromolecules 18,2454-2462 (2017).
- Ma, L., Gaisinskaya-Kipnis, A., Kampf, N. & Klein, J. Origins of hydration lubrication. Nature Communications 6,6060 (2015).
- Biegler, M., Delius, J., Käsdorf, B. T., Hofmann, T. & Lieleg, O. Cationic astringents alter the tribological and rheological properties of human saliva and salivary mucin solutions. Biotribology 6,12-20 (2016).
- Winkeljann, B., Boettcher, K., Balzer, B. N. & Lieleg, O. Mucin Coatings Prevent Tissue Damage at the Cornea-Contact Lens Interface. Advanced Materials Interfaces 4, 1700186 (2017).
- Boettcher, K., Winkeljann, B., Schmidt, T. A. & Lieleg, O. Quantification of cartilage wear morphologies in unidirectional sliding experiments: Influence of different macromolecular lubricants. Biotribology (2017).
- Kocevar-Nared, J., Kristl, J., Smid-Korbar, J. Comparative rheological investigation of crude gastric mucin and natural gastric mucus. Biomaterials 18,677-681 (1997).
- Schömig, V. J. et al. An optimized purification process for porcine gastric mucin with preservation of its native functional properties. RSC Advances 6,44932-44943 (2016).

## Claims

1. A method of isolating mucin from a mucin containing tissue, wherein the method comprises subjecting a mucin containing solution to at least two subsequent filtration steps with no centrifugation step, wherein the mucin containing solution has been produced by contacting a mucin containing tissue with an aqueous solution over a suitable period of time, which is at least about 10 min, at a suitable temperature, which is in the range of about 0°C to about 42°C, for transferring the mucin into the aqueous solution, wherein the aqueous solution has an osmolarity of at least about 800 mOsm/L, prior to the step of contacting the mucin containing tissue with the aqueous solution, a pore size diameter of a first filter used in a first step and a pore size diameter of a second filter used in a second step are different, and wherein the second filter used in the second step has a smaller pore size diameter than the first filter used in the first step, wherein the mucin containing solution is filtered in the first filtration step by using the first filter having the pore size diameter in the range of about 3 mm to about 0.5 mm, wherein the mucin containing solution is filtered in the second filtration step by using the pore size diameter in the range of about 600 µm to about 10 µm, thereby producing a filtered mucin containing solution.

2. The method of claim 1, wherein contacting the mucin containing tissue with the aqueous solution comprises manually scraping, brushing, or washing the mucin from the mucin containing tissue into the aqueous solution, or adding the aqueous solution to the mucin containing tissue.

3. The method of claim 1, wherein contacting comprises adding the aqueous solution to the mucin containing tissue in an aerated bubble column.

4. The method of any one of the preceding claims, wherein the mucin containing solution is filtered in the first filtration step by using the first filter having the pore size diameter of about 1 mm.

5. The method of claim 4, wherein a mesh is used as the first filter for the first filtration step, preferably wherein the mesh is made of any one of nitrocellulose, mixed cellulose ester (MCE), glass, glass fiber, glass foam, plastic, polypropylene (PP), polyether sulfone (PES), polytetrafluoroethylene (PTFE), polyvinylidene fluoride (PVDF), nylon, tissue, felt, wool, fleece or metal, preferably a stainless steel mesh.

6. The method of any one of the preceding claims, wherein the mucin containing solution is filtered in the second filtration step by using the second filter having the pore size diameter of about 100 µm.

7. The method of claim 6, wherein a filter is used as the second filter for the second filtration step, preferably wherein the filter is a paper filter, a nitrocellulose filter, a mixed cellulose ester (MCE) filter, a glass filter, a glass fiber filter, a glass foam filter, a syringe filter, a filter sleeve, a sterile filter unit, a plastic filter, a polypropylene (PP) filter, a polyethylene sulfone (PES) filter, a polytetrafluoroethylene (PTFE) filter, a polyvinylidene fluoride (PVDF) filter, a tissue filter, a felt filter, a wool filter, a fleece filter, or a metal filter, preferably a stainless steel filter.

8. The method of any one of the preceding claims, further comprises filtering the mucin containing solution in a third filtration step, preferably wherein each of the filtration steps is repeated at least once before the next subsequent filtration step is performed.

9. The method of any one of the preceding claims, wherein the mucin containing tissue is of plant or animal origin.

10. The method of any one of the preceding claims, wherein the pH of the aqueous solution is in a range of pH 4 to pH 9, prior to the step of contacting the mucin containing tissue with the aqueous solution, preferably wherein the pH is approximately 7.

11. The method of any one of the preceding claims, wherein the aqueous solution is contacted with the mucin containing tissue over a suitable period of time which is at least about 30 min, or at least about 1 h, and preferably is a saline solution or a saline buffer.

12. The method of any one of the preceding claims, wherein the filtered mucin containing solution is subjected to a size exclusion chromatography, thereby producing a mucin enriched solution, and preferably wherein the mucin enriched solution is subjected to diafiltration with demineralized, distilled or ultrapure water.

## Patentansprüche

1. Verfahren zum Isolieren von Mucin aus einem Mucin enthaltenden Gewebe, wobei das Verfahren das Unterziehen einer Mucin enthaltenden Lösung mindestens zwei aufeinanderfolgenden Filtrationsschritten ohne Zentrifugationsschritt umfasst, wobei die Mucin enthaltende Lösung durch Inkontaktbringen eines Mucin enthaltenden Gewebes mit einer wässrigen Lösung über einen geeigneten Zeitraum, der mindestens etwa 10 min beträgt, bei einer geeigneten Temperatur, die im Bereich von etwa 0 °C bis etwa 42 °C liegt, zum Überführen des Mucins in die wässrige Lösung hergestellt wurde, wobei die wässrige Lösung eine Osmolarität von mindestens etwa 800 mOsm/L aufweist, vor dem Schritt des Inkontaktbringens des Mucin enthaltenden Gewebes mit der wässrigen Lösung ein Porengrößendurchmesser eines ersten Filters, der in einem ersten Schritt verwendet wird, und ein Porengrößendurchmesser eines zweiten Filters, der in einem zweiten Schritt verwendet wird, unterschiedlich sind, und wobei der zweite Filter, der in dem zweiten Schritt verwendet wird, einen kleineren Porengrößendurchmesser als der erste Filter, der in dem ersten Schritt verwendet wird, aufweist, wobei die Mucin enthaltende Lösung in dem ersten Filtrationsschritt unter Verwendung des ersten Filters mit dem Porengrößendurchmesser im Bereich von etwa 3 mm bis etwa 0,5 mm gefiltert wird, wobei die Mucin enthaltende Lösung in dem zweiten Filtrationsschritt unter Verwendung des Porengrößendurchmessers im Bereich von etwa 600 µm bis etwa 10 µm gefiltert wird, wodurch eine gefilterte Mucin enthaltende Lösung hergestellt wird.

2. Verfahren nach Anspruch 1, wobei das Inkontaktbringen des Mucin enthaltenden Gewebes mit der wässrigen Lösung das manuelle Schaben, Bürsten oder Waschen des Mucins aus dem Mucin enthaltenden Gewebe in die wässrige Lösung oder das Zugeben der wässrigen Lösung zu dem Mucin enthaltenden Gewebe umfasst.

3. Verfahren nach Anspruch 1, wobei das Inkontaktbringen das Zugeben der wässrigen Lösung zu dem Mucin enthaltenden Gewebe in einer belüfteten Blasensäule umfasst.

4. Verfahren nach einem der vorhergehenden Ansprüche, wobei die Mucin enthaltende Lösung in dem ersten Filtrationsschritt unter Verwendung des ersten Filters mit dem Porengrößendurchmesser von etwa 1 mm gefiltert wird.

5. Verfahren nach Anspruch 4, wobei ein Netz als der erste Filter für den ersten Filtrationsschritt verwendet wird, wobei das Netz vorzugsweise aus einem von Nitrocellulose, gemischtem Celluloseester (MCE), Glas, Glasfaser, Glasschaum, Kunststoff, Polypropylen (PP), Polyethersulfon (PES), Polytetrafluorethylen (PTFE), Polyvinylidenfluorid (PVDF), Nylon, Gewebe, Filz, Wolle, Vlies oder Metall, vorzugsweise einem Edelstahlnetz, hergestellt ist.

6. Verfahren nach einem der vorhergehenden Ansprüche, wobei die Mucin enthaltende Lösung in dem zweiten Filtrationsschritt unter Verwendung des zweiten Filters mit dem Porengrößendurchmesser von etwa 100 µm gefiltert wird.

7. Verfahren nach Anspruch 6, wobei ein Filter als der zweite Filter für den zweiten Filtrationsschritt verwendet wird, wobei der Filter vorzugsweise ein Papierfilter, ein Nitrocellulosefilter, ein gemischter Celluloseester (MCE)-Filter, ein Glasfilter, ein Glasfaserfilter, ein Glasschaumfilter, ein Spritzenfilter, eine Filterhülse, eine sterile Filtereinheit, ein Kunststofffilter, ein Polypropylen (PP)-Filter, ein Polyethylensulfon (PES)-Filter, ein Polytetrafluorethylen (PTFE)-Filter, ein Polyvinylidenfluorid (PVDF)-Filter, ein Gewebefilter, ein Filzfilter, ein Wollefilter, ein Vliesfilter oder ein Metallfilter, vorzugsweise ein Edelstahlfilter, ist.

8. Verfahren nach einem der vorhergehenden Ansprüche, das ferner das Filtern der Mucin enthaltenden Lösung in einem dritten Filtrationsschritt umfasst, wobei vorzugsweise jeder der Filtrationsschritte mindestens einmal wiederholt wird, bevor der nächste nachfolgende Filtrationsschritt durchgeführt wird.

9. Verfahren nach einem der vorhergehenden Ansprüche, wobei das Mucin enthaltende Gewebe pflanzlichen oder tierischen Ursprungs ist.

10. Verfahren nach einem der vorhergehenden Ansprüche, wobei der pH-Wert der wässrigen Lösung vor dem Schritt des Inkontaktbringens des Mucin enthaltenden Gewebes mit der wässrigen Lösung in einem Bereich von pH 4 bis pH 9 liegt, wobei der pH-Wert vorzugsweise etwa 7 beträgt.

11. Verfahren nach einem der vorhergehenden Ansprüche, wobei die wässrige Lösung mit dem Mucin enthaltenden Gewebe über einen geeigneten Zeitraum in Kontakt gebracht wird, der mindestens etwa 30 min oder mindestens etwa 1 h beträgt und vorzugsweise eine Kochsalzlösung oder ein Kochsalzlösung-Puffer ist.

12. Verfahren nach einem der vorhergehenden Ansprüche, wobei die gefilterte Mucin enthaltende Lösung einer Größenausschlusschromatographie unterzogen wird, wodurch eine Mucin-angereicherte Lösung erzeugt wird, und wobei vorzugsweise die Mucin-angereicherte Lösung einer Diafiltration mit demineralisiertem, destilliertem oder ultrareinem Wasser unterzogen wird.

## Revendications

1. Procédé d'isolement de mucine à partir d'un tissu contenant de la mucine, dans lequel le procédé comprend la soumission d'une solution contenant de la mucine à au moins deux étapes de filtration ultérieures sans étape de centrifugation, dans lequel la solution contenant de la mucine a été produite par mise en contact d'un tissu contenant de la mucine avec une solution aqueuse sur une période de temps appropriée, qui est d'au moins environ 10 min, à une température appropriée, qui est dans la plage d'environ 0 °C à environ 42 °C, pour transférer la mucine dans la solution aqueuse, dans lequel la solution aqueuse a une osmolarité d'au moins environ 800 mOsm/L, avant l'étape de mise en contact du tissu contenant de la mucine avec la solution aqueuse, un diamètre de taille de pore d'un premier filtre utilisé dans une première étape et un diamètre de taille de pore d'un second filtre utilisé dans une seconde étape sont différents, et dans lequel le second filtre utilisé dans la seconde étape a un diamètre de taille de pore plus petit que le premier filtre utilisé dans la première étape, dans lequel la solution contenant de la mucine est filtrée dans la première étape de filtration en utilisant le premier filtre ayant le diamètre de taille de pore dans la plage d'environ 3 mm à environ 0,5 mm, dans lequel la solution contenant de la mucine est filtrée dans la seconde étape de filtration en utilisant le diamètre de taille de pore dans la plage d'environ 600 µm à environ 10 µm, produisant ainsi une solution contenant de la mucine filtrée.

2. Procédé selon la revendication 1, dans lequel la mise en contact du tissu contenant de la mucine avec la solution aqueuse comprend le raclage manuel, le brossage, ou le lavage de la mucine à partir du tissu contenant de la mucine dans la solution aqueuse, ou l'ajout de la solution aqueuse au tissu contenant de la mucine.

3. Procédé selon la revendication 1, dans lequel la mise en contact comprend l'ajout de la solution aqueuse au tissu contenant de la mucine dans une colonne à bulles aérée.

4. Procédé selon l'une quelconque des revendications précédentes, dans lequel la solution contenant de la mucine est filtrée dans la première étape de filtration en utilisant le premier filtre ayant le diamètre de taille de pore d'environ 1 mm.

5. Procédé selon la revendication 4, dans lequel une maille est utilisée en tant que premier filtre pour la première étape de filtration, de préférence dans lequel la maille est constituée de l'un quelconque parmi la nitrocellulose, un ester de cellulose mixte (MCE), le verre, la fibre de verre, la mousse de verre, le plastique, le polypropylène (PP), la polyéthersulfone (PES), le polytétrafluoroéthylène (PTFE), le fluorure de polyvinylidène (PVDF), le nylon, le tissu, le feutre, la laine, la polaire ou le métal, de préférence une maille en acier inoxydable.

6. Procédé selon l'une quelconque des revendications précédentes, dans lequel la solution contenant de la mucine est filtrée dans la seconde étape de filtration en utilisant le second filtre ayant le diamètre de taille de pore d'environ 100 µm.

7. Procédé selon la revendication 6, dans lequel un filtre est utilisé en tant que second filtre pour la seconde étape de filtration, de préférence dans lequel le filtre est un filtre en papier, un filtre en nitrocellulose, un filtre en ester de cellulose mixte (MCE), un filtre en verre, un filtre en fibre de verre, un filtre en mousse de verre, un filtre à seringue, un manchon de filtre, une unité de filtre stérile, un filtre en plastique, un filtre en polypropylène (PP), un filtre en polyéthylènesulfone (PES), un filtre en polytétrafluoroéthylène (PTFE), un filtre en fluorure de polyvinylidène (PVDF), un filtre en tissu, un filtre en feutre, un filtre en laine, un filtre en polaire, ou un filtre en métal, de préférence un filtre en acier inoxydable.

8. Procédé selon l'une quelconque des revendications précédentes, comprenant en outre la filtration de la solution contenant de la mucine dans une troisième étape de filtration, de préférence dans lequel chacune des étapes de filtration est répétée au moins une fois avant que l'étape de filtration suivante ne soit effectuée.

9. Procédé selon l'une quelconque des revendications précédentes, dans lequel le tissu contenant de la mucine est d'origine végétale ou animale.

10. Procédé selon l'une quelconque des revendications précédentes, dans lequel le pH de la solution aqueuse est dans une plage de pH 4 à pH 9, avant l'étape de mise en contact du tissu contenant de la mucine avec la solution aqueuse, de préférence dans lequel le pH est d'environ 7.

11. Procédé selon l'une quelconque des revendications précédentes, dans lequel la solution aqueuse est mise en contact avec le tissu contenant de la mucine sur une période de temps appropriée qui est d'au moins environ 30 min, ou d'au moins environ 1 h, et est de préférence une solution saline ou un tampon salin.

12. Procédé selon l'une quelconque des revendications précédentes, dans lequel la solution contenant de la mucine filtrée est soumise à une chromatographie d'exclusion stérique, produisant ainsi une solution enrichie en mucine, et de préférence dans lequel la solution enrichie en mucine est soumise à une diafiltration avec de l'eau déminéralisée, distillée ou ultrapure.
